# FASCICULE DE BREVET EUROPEEN

(11) **EP 1 408 889 B1**
(45) Date de publication et mention de la délivrance du brevet: **30.06.2010**
(21) Numéro de dépôt: 01993439.7
(22) Date de dépôt: 31.10.2001
(51) Int. Cl.: A61F 2/44, A61F 2/46

(54) **CAGE INTERVERTEBRALE POUR METTRE EN PLACE ENTRE DEUX VERTEBRES**
ZWISCHENWIRBELKÄFIG ZUM EINSETZEN ZWISCHEN ZWEI WIRBEL
INTERVERTEBRAL CAGE FOR SETTING BETWEEN TWO VERTEBRAE

(30) Priorité: 07.11.2000 FR 0014264
(43) Date de publication de la demande: 21.04.2004
(73) Titulaire: Medicrea, 17000 La Rochelle (FR)
(72) Inventeur: ADAM, Yves, F-14280 AUTHIE (FR); VILLARET, Bernard, F-17220 CROIX CHAPEAU (FR); BARBERA, José-Vicente, 46009 VALENCIA (ES)
(74) Mandataire: Jeannet, Olivier
(86) Numéro de dépôt international: PCT/FR2001/003391
(87) Numéro de publication internationale: WO 2002/038086

(56) Documents cités:
- EP-A- 0 834 295
- WO-A-95/08306
- FR-A- 2 726 759
- FR-A- 2 764 795
- FR-A- 2 782 632
- US-A- 6 080 158

## Description

La présente invention concerne une cage intervertébrale destinée à être mise en place entre deux vertèbres.

Une cage de ce type est utilisée pour rétablir la distance anatomique de deux vertèbres lorsque le disque reliant ces vertèbres est affaissé, et pour opérer une immobilisation relative de ces vertèbres par croissance osseuse dans l'espace intervertébral.

Pour la mise en place d'une telle cage, un logement approprié est aménagé au travers du disque et dans les plateaux vertébraux, les vertèbres sont distractées puis la cage est insérée dans le logement au moyen d'un instrument de mise en place et d'impaction.

Les cages existantes présentent généralement des formes cylindriques, tronconiques ou parallélépipédiques. Elles ont des dimensions relativement importantes dans le sens de leur épaisseur afin de délimiter des surfaces de contact relativement étendues avec les vertèbres. Ces surfaces ont pour but de prévenir au maximum le risque d'insertion des cages dans les vertèbres, résultant des sollicitations importantes et répétées que les vertèbres exercent sur elles. Les logements de réception de ces cages doivent eux-mêmes avoir des dimensions relativement importantes, de sorte que leur aménagement implique une ablation d'os conséquente, plutôt délicate à réaliser compte tenu de la proximité de la moelle épinière et des terminaisons nerveuses.

En outre, certaines cages d'épaisseur relativement importante occupent un espace non négligeable et conduisent par conséquent à un risque que la fusion des vertèbres ne soit pas parfaitement solide.

Les documents n° WO 95/08306 ou US 6 080 158 décrivent chacun une cage présentant une forme allongée et délimitant quatre faces latérales, à savoir deux faces latérales principales, placées parallèlement à l'axe de la colonne vertébrale lorsque la cage est mise en place et définissant entre elles l'épaisseur de la cage, et deux faces latérales secondaires, reliant les deux faces principales l'une à l'autre et destinées à venir en contact avec les vertèbres ; lesdites faces secondaires présentent, vues selon une direction perpendiculaire auxdites faces principales, une courbure de forme convexe correspondant à la forme concave que présentent les plateaux vertébraux dans le plan antéro-postérieur, et l'épaisseur de la cage est réduite, c'est-à-dire peut aller de 3,5 à 7 mm.

Les cages selon la technique antérieure ont pour inconvénients de présenter des risques de basculement non négligeables, particulièrement lorsqu'elles présentent des épaisseurs réduites, ainsi que des risques d'insertion douloureuse dans les vertèbres sous l'effet de la charge qui leur est transmise.

La présente invention vise à remédier à ces inconvénients essentiels.

Son but de fournir une cage intervertébrale peu invasive, c'est-à-dire limitant les opérations à accomplir pour l'aménagement du logement de réception de cette cage, présentant un risque limité d'insertion dans les plateaux vertébraux et garantissant l'obtention d'une fusion parfaitement solide des vertèbres.

La cage concernée est du type de celle connue par les documents antérieurs précités n° WO 95/08306 ou US 6 080 158.

Selon l'invention, la cage présente, au niveau d'au moins une desdites faces secondaires, une rainure longitudinale unique aménagée dans la zone médiane de cette face secondaire, et la cage a une forme convexe au niveau de cette même face, vue dans la direction longitudinale de la cage.

Cette rainure et cette forme convexe permettent d'aménager deux zones d'appui décalées latéralement, qui favorisent la stabilité de la cage contre un risque de basculement. De plus, la rainure permet d'aménager un espace longitudinal pouvant recevoir des copeaux d'os ou pouvant être envahi par des cellules osseuses lors de leur croissance, ce qui permet de constituer une nervure osseuse apte à parfaitement caler la cage par rapport aux vertèbres. En outre, la courbure convexe desdites faces secondaires augmente la surface de ces faces et supprime l'existence d'un angle saillant entre ces faces secondaires et les faces principales de la cage, ce qui favorise un appui non agressif de la cage contre les plateaux vertébraux et réduit ainsi le risque d'insertion douloureuse de la cage dans les vertèbres.

De préférence, la cage présente la rainure et la forme convexe précitées au niveau de ses deux faces secondaires.

Avantageusement, la cage présente au moins une série d'entailles au niveau d'au moins une de ses faces secondaires, ces entailles étant conformées pour délimiter des dents favorisant une légère insertion de la cage dans les plateaux vertébraux.

Cette légère insertion contribue à favoriser le maintien de la cage en position par rapport aux vertèbres le temps que s'opère la croissance des cellules osseuses.

Dans le même but, au moins une des parois principales et/ou secondaires de la cage peut comprendre au moins un trou destiné à être envahi par les cellules osseuses lors de leur croissance.

Avantageusement, la cage est percée longitudinalement d'un trou qui débouche dans l'une de ses faces d'extrémité, et comprend des cannelures aménagées dans la paroi qui délimite ce trou.

Ce trou et ces cannelures sont destinés à recevoir l'extrémité de forme correspondante de l'instrument de mise en place de la cage. La profondeur de ce trou et la présence de ces cannelures permettent une parfaite immobilisation de la cage par rapport à l'instrument de mise en place afin de permettre un positionnement fiable de la cage par rapport au logement de réception lors de l'insertion et de l'impaction de cette cage dans ce logement.

Selon une forme de réalisation préférée de l'invention, la cage présente un alésage taraudé dans le fond dudit trou, coaxial à ce trou.

Cet alésage est destiné à recevoir l'extrémité filetée d'une tige que comprend ledit instrument de mise en place, cette tige permettant une extraction de la cage si nécessaire. Il est ainsi rendu possible de tester l'implantation de la cage afin de vérifier si le logement de réception est correctement aménagé.

En conséquence de ce qui précède, l'instrument de mise en place de la cage comprend deux pièces, à savoir :
- une pièce tubulaire, dont une extrémité comprend une forme correspondant à celle dudit trou et desdites cannelures, et
- une tige engagée et pouvant coulisser dans cette pièce tubulaire, dont une extrémité comprend un filetage permettant son engagement dans ledit alésage taraudé.

Pour sa bonne compréhension, l'invention est à nouveau décrite ci-dessous en référence au dessin schématique annexé représentant, à titre d'exemple non limitatif, une forme de réalisation préférée de la cage intervertébrale et de l'instrument de mise en place de cette cage, qu'elle concerne.
La figure 1 est une vue en perspective de la cage ;
la figure 2 en est une vue de côté ;
la figure 3 en est une vue en bout ;
les figures 4 et 5 en sont des vues après mise en place entre deux vertèbres, respectivement latéralement et du côté dorsal, et

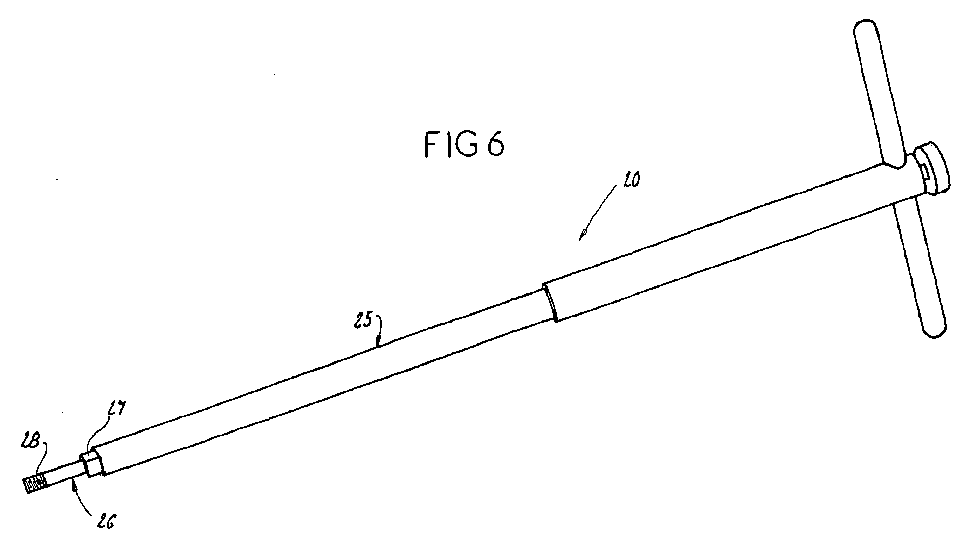

la figure 6 est une vue de côté de l'instrument.

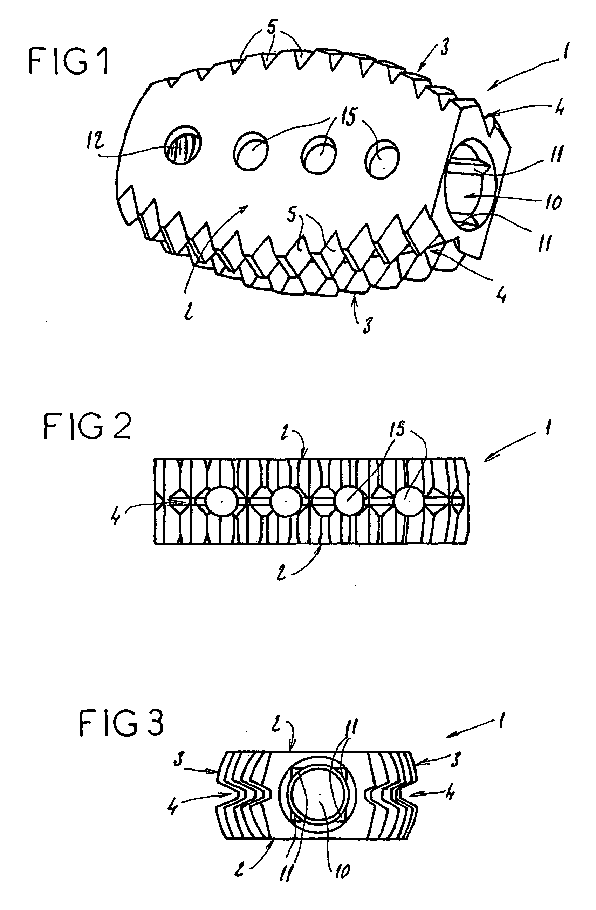

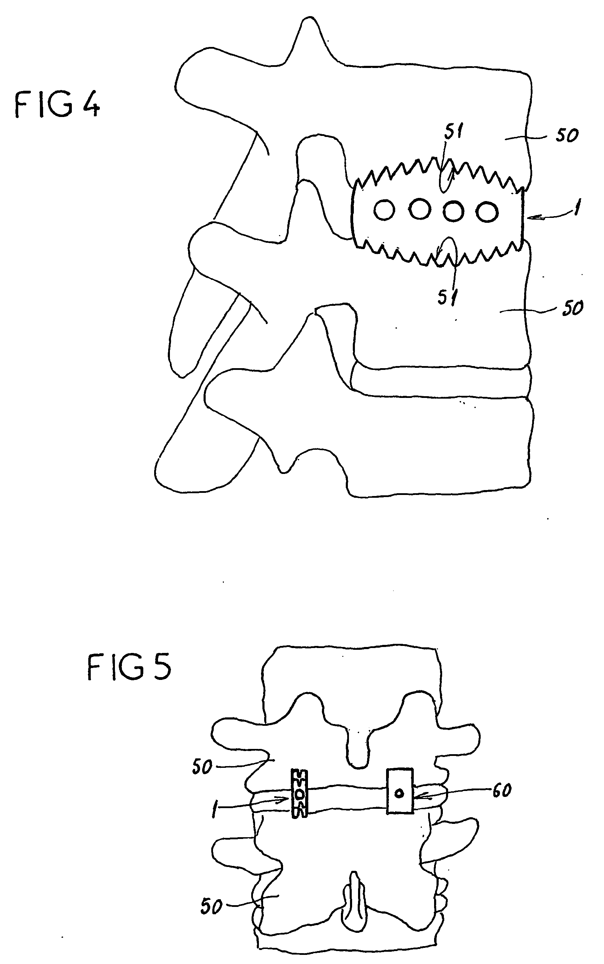

Les figures 1 à 3 représentent sous différents angles une cage intervertébrale 1, destinée, ainsi que le montrent les figures 4 et 5, à rétablir la distance anatomique de deux vertèbres 50 lorsque le disque reliant ces vertèbres 50 est affaissé, et pour opérer une immobilisation relative de ces vertèbres 50 par croissance osseuse dans l'espace intervertébral.

La cage 1 présente une forme allongée et délimite quatre faces latérales 2, 3, à savoir deux faces latérales principales 2 et deux faces latérales secondaires 3.

Les faces 2 sont planes et destinées à être placées parallèlement à l'axe de la colonne vertébrale lorsque la cage 1 est mise en place. Elles définissent entre elles l'épaisseur de la cage 1, qui est relativement réduite, à savoir qui correspond à environ le quart de la longueur de la cage 1. En particulier, la cage 1 peut avoir une longueur de 20 mm et donc une épaisseur de l'ordre de 5 mm.

Les faces 3 relient les faces 2 l'une à l'autre et sont destinées à venir en contact avec les vertèbres 50. Comme le montre la figure 4, ces faces 3 présentent, lorsqu'elles sont vues selon une direction perpendiculaire aux faces 2, une courbure de forme convexe correspondant à la forme concave que présentent les plateaux vertébraux 51 dans le plan antéro-postérieur. Ces faces 3, lorsqu'elles sont vues dans la direction longitudinale de la cage (cf. figure 3) ont de plus une forme courbe, et la cage 1 comprend deux rainures longitudinales 4, à section en forme de "V", qui débouchent dans la zone médiane de chacune d'elles.

La cage 1 présente quatre séries d'entailles 5 aménagées dans ses parois délimitées par les faces 3. Ces entailles 5 ont une forme de "V" à angle de 45°, et sont conformées pour délimiter des dents émoussées favorisant une légère insertion de la cage 1 dans les plateaux 51.

La cage 1 est en outre percée longitudinalement d'un trou 10 qui débouche dans la face proximale d'extrémité. Elle comprend quatre cannelures proximales 11 aménagées dans la paroi qui délimite ce trou 10, ces cannelures définissant les angles d'un carré comme le montre la figure 3. Les cannelures 11 permettent le calage de la cage 1 sur l'extrémité de l'instrument de pose comme cela apparaîtra plus loin. La cage comprend également un alésage taraudé 12 dans le fond du trou 10, coaxial à ce dernier.

La cage 1 présente en outre des trous 15 traversant ses parois latérales 2, 3 et mettant le trou 10 en communication avec l'extérieur.

Les dimensions de la cage 1 autres que celles indiquées plus haut peuvent en particulier être les suivantes : hauteur : 9, 11 ou 13 mm (trois modèles étant prévus pour adapter la hauteur de la cage aux différentes hauteurs d'espace intervertébral possibles), diamètre générant la courbure des faces 3 dans le sens transversal de la cage : 12,91 mm, diamètre du trou 10 : 3,5 mm, diamètre des trous 15 : 2 mm.

L'instrument 20 de mise en place de la cage 1 comprend quant à lui deux pièces 25, 26, à savoir :
- une pièce tubulaire 25, dont une extrémité présente une zone de forme carrée 27 dont la forme correspond à l'espace carré délimité par les cannelures 11, et
- une tige 26 engagée et pouvant coulisser dans cette pièce tubulaire 25, dont une extrémité 28 comprend un filetage permettant son engagement dans l'alésage taraudé 12.

En pratique, deux cages 1 sont la plupart du temps mises en place au niveau d'un même disque intervertébral, de part et d'autre de la colonne vertébrale. Pour ce faire, comme cela se déduit de la figure 5, deux logements de réception sont aménagés au travers du disque intervertébral, sans résection des plateaux vertébraux 51. Les vertèbres 50 sont ensuite distractées au moyen d'une cale 60 insérée dans l'un des logements puis une cage 1 est insérée dans l'autre logement au moyen de l'instrument 20.

Après mise en place de la première cage 1, la cale 60 est retirée ; la seconde cage 1 est alors mise en place dans le second logement avec bourrage de copeaux d'os spongieux autour d'elle.

Comme indiqué précédemment, l'invention fournit une cage intervertébrale présentant, par rapport aux cages homologues de la technique antérieure, l'avantage essentiel d'être à la fois peu invasive, c'est-à-dire de limiter les opérations à accomplir pour l'aménagement du logement de réception de cette cage, de présenter un risque limité d'insertion dans les plateaux vertébraux et de garantir l'obtention d'une fusion parfaitement solide des vertèbres.

Il va de soi que l'invention n'est pas limitée à la forme de réalisation décrite ci-dessus à titre d'exemple mais qu'elle en embrasse au contraire toutes les variantes de réalisation visées par les revendications ci-annexées.

## Revendications

1. Cage intervertébrale, présentant une forme allongée et délimitant quatre faces latérales (2,3), à savoir deux faces latérales principales (2), placées parallèlement à l'axe de la colonne vertébrale lorsque la cage (1) est mise en place et définissant entre elles -l'épaisseur de la cage, et deux faces latérales secondaires (3), reliant les deux faces principales (2) l'une à l'autre et destinées à venir en contact avec les vertèbres (50) ; lesdites faces secondaires (3) présentent, vues selon une direction perpendiculaire auxdites faces principales (2), une courbure de forme convexe correspondant à la forme concave que présentent les plateaux vertébraux (51) dans le plan antéro-postérieur, et l'épaisseur de la cage (1) est réduite, c'est-à-dire entre 3,5 et 7 mm, la cage présentant, au niveau d'au moins une desdites faces secondaires (3), une rainure longitudinale (4),
cage **caractérisée en ce que** la rainure longitudinale (4) est unique et est aménagée dans la zone médiane de ladite face secondaire (3), et **en ce que** la cage a une forme convexe au niveau de cette même face secondaire (3), vue dans la direction longitudinale de la cage (1).

2. - Cage intervertébrale selon la revendication 1, **caractérisée en ce qu'**elle présente ladite rainure longitudinale (4) et ladite forme courbe au niveau de ses deux faces secondaires (3).

3. - Cage intervertébrale selon la revendication 1 ou la revendication 2, **caractérisée en ce qu'**elle présente au moins une série d'entailles (5) au niveau d'au moins une de ses faces secondaires (3), ces entailles (5) étant conformées pour délimiter des dents favorisant une légère insertion de la cage (1) dans les plateaux vertébraux (51).

4. - Cage intervertébrale selon l'une des revendications 1 à 3, **caractérisée en ce qu'**au moins une de ses parois principales et/ou secondaires comprend au moins un trou (15) destiné à être envahi par les cellules osseuses lors de leur croissance.

5. - Cage intervertébrale selon l'une des revendications 1 à 4, **caractérisée en ce qu'**elle est percée longitudinalement d'un trou (10) qui débuche dans l'une de ses faces d'extrémité, et **en ce qu'**elle comprend des cannelures (11) aménagées dans la paroi qui délimite ce trou (10).

6. - Cage intervertébrale selon la revendication 5, **caractérisée en ce qu'**elle présente un alésage taraudé (12) dans le fond dudit trou (10), coaxial à ce trou (10).

## Claims

1. - Intervertebral cage, having an elongated shape and defining four lateral faces (2, 3), namely two main lateral faces (2), arranged parallel to the axis of the spine when the cage (1) is implanted defining between them the thickness of the cage, and two secondary lateral faces (3), linking the two main faces (2) one another and for making contact with the vertebrae (50); such secondary faces (3) present, when seen in a direction perpendicular to said main faces (2), a convex curvature corresponding to the concave shape that have the plates (51) in the antero-posterior plane, and the thickness of the cage (1) is reduced, that is to say between 3.5 and 7 mm, with the cage having, in at least one of said secondary faces (3), a longitudinal groove (4);
cage **characterized in that** the longitudinal groove (4) is unic and is arranged in the middle of said secondary face (3), and that the cage has a convex shape at this same secondary face (3), seen in the longitudinal direction of the cage (1).

2. - Intervertebral cage according to claim 1, **characterized in that** it has said longitudinal groove (4) and said convex shape at its two side faces (3).

3. - Intervertebral cage according to claim 1 or claim 2, **characterized in that** it has at least one series of notches (5) in at least one of its lateral faces (3), these notches (5) being shaped so as to define teeth promoting a slight insertion of the cage (1) in the vertebral plates (51).

4. - Intervertebral cage according to claim 1 to 3, **characterized in that** at least one of its main faces and/or side faces includes at least one hole (15) to be invaded by bone cells during their growth.

5. - Intervertebral cage according to claim 1 to 4, **characterized in that** it is longitudinally bored by a hole (10) which opens into one of its end faces, and **in that** it includes grooves (11) arranged in the wall that delimits this hole (10).

6. - Intervertebral cage according to claim 5, **characterized in that** it has a tapped hole (12) in the bottom of said hole (10), coaxial to this hole (10).

## Patentansprüche

1. Zwischenwirbelkäfig, der eine längliche Form aufweist und vier Seitenflächen (2, 3) begrenzt, nämlich zwei Hauptseitenflächen (2), die parallel zur Achse der Wirbelsäule platziert sind, wenn der Käfig (1) platziert ist und die zwischen sich die Dicke des Käfigs definieren, und zwei sekundäre Seitenflächen (3), die die zwei Hauptseitenflächen (2) miteinander verbinden und dazu bestimmt sind, mit den Wirbeln (50) in Kontakt zu kommen, wobei die sekundären Flächen (3), wenn sie in senkrechter Richtung zu den Hauptflächen (2) betrachtet werden, eine konvexe Krümmung aufweisen, die der konkaven Form entspricht, die die Wirbelplatten (51) in anteroposteriorer Ebene aufweisen, und die Dicke des Käfigs (1) reduziert ist, das heißt zwischen 3,5 und 7 mm, wobei der Käfig auf Ebene mindestens einer der sekundären Flächen (3) eine Längskerbe (4) aufweist,
wobei der Käfig **dadurch gekennzeichnet ist, dass** die Längskerbe (4) einzig ist und in den mittleren Bereich der sekundären Fläche (3) eingearbeitet ist, und **dadurch**, dass der Käfig, betrachtet in Längsrichtung des Käfigs (1), auf Ebene derselben sekundären Fläche (3) eine konvexe Form hat.

2. Zwischenwirbelkäfig nach Anspruch 1, **dadurch gekennzeichnet, dass** er die Längskerbe (4) und die gekrümmte Form auf Ebene seiner zwei sekundären Flächen (3) aufweist.

3. Zwischenwirbelkäfig nach Anspruch 1 oder Anspruch 2, **dadurch gekennzeichnet, dass** er mindestens eine Reihe Einkerbungen (5) auf Ebene mindestens einer seiner sekundären Flächen (3) aufweist, wobei diese Einkerbungen (5) ausgebildet sind, um Zähne zu begrenzen, die ein problemloses Einsetzen des Käfigs (1) in die Wirbelplatten (51) erlaubt.

4. Zwischenwirbelkäfig nach einem der Ansprüche 1 bis 3, **dadurch gekennzeichnet, dass** mindestens eine der Haupt- und/oder sekundären Flächen mindestens ein Loch (15) umfasst, das dazu bestimmt ist, von Knochenzellen während ihres Wachstums belegt zu werden.

5. Zwischenwirbelkäfig nach einem der Ansprüche 1 bis 4, **dadurch gekennzeichnet, dass** er längs von einem Loch (10) durchbohrt ist, das in eine seiner Endflächen mündet, und **dadurch**, dass er Riefen (11) umfasst, die in die dieses Loch (10) begrenzende Wand eingearbeitet sind.

6. Zwischenwirbelkäfig nach Anspruch 5, **dadurch gekennzeichnet, dass** er im Grund dieses Lochs (10) eine Gewindebohrung (12) aufweist, die zu diesem Loch (10) koaxial ist.
